(19) Europäisches Patentamt
European Patent Office
Office européen des brevets

(11) Publication number: **0 197 756**
**A2**

(12) **EUROPEAN PATENT APPLICATION**

(21) Application number: 86302435.2

(22) Date of filing: 02.04.86

(51) Int. Cl.[4]: C 07 C 103/375
C 07 C 103/38, C 07 C 147/12
C 07 D 265/30, C 07 D 213/75
C 07 C 121/78, A 01 N 37/22
A 01 N 37/34, A 01 N 43/40
A 01 N 43/84, A 01 N 37/24

(30) Priority: 05.04.85 US 720236

(43) Date of publication of application:
15.10.86 Bulletin 86/42

(84) Designated Contracting States:
AT BE CH DE FR GB IT LI LU NL SE

(71) Applicant: ELI LILLY AND COMPANY
Lilly Corporate Center
Indianapolis Indiana 46285(US)

(72) Inventor: Gajewski, Robert Peter
1501 Friendship Drive
Indianapolis Indiana 46217(US)

(74) Representative: Tapping, Kenneth George et al,
Erl Wood Manor
Windlesham Surrey, GU20 6PH(GB)

(54) **Alkanoyl anilides.**

(57) The present invention is an alkanoyl anilides exhibiting insecticidal and arachnicidal activity, wherein the alkanoyl is both branched and substantially or totally fluorinated.

EP 0 197 756 A2

Croydon Printing Company Ltd.

# ALKANOYL ANILIDES

The present invention is in alkanoyl anilides of which the alkanoyl is both branched and substantially or totally fluorinated. These anilides exhibit excellent insecticidal and arachnicidal activity. The invention therefore also includes methods employing, and formulations comprising, these compounds as insecticides and arachnicides.

More specifically, the invention provides compounds of the formulae:

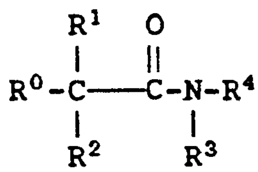

I

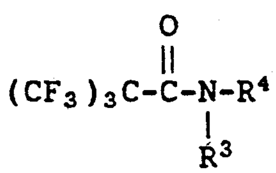

II and

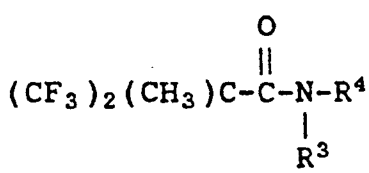

III

wherein $R^0$ represents

bromo,

chloro, or

fluoro;

$R^1$ represents

$CF_3$,

$C_2F_5$,

$C_3F_7$, or

a n-, iso-, or sec-$C_4F_9$;

$R^2$ represents

$CF_3$, $C_2F_5$, $C_3F_7$, or,

when $R^0$ represents fluoro, $R^2$ can additionally represent

$-OR_f$,

$-N(R_f)_2$,

-CN,

$-CF_2-OR_f$, or

$-CF_2-N(R_f)_2$

and each $R_f$ independently represents perfluoro-loweralkyl of $C_1$-$C_3$ or, in $-N(R_f)_2$ both $R_f$ groups can be taken together with the N and constitute perfluoropyrrolidino, perfluoropiperidino, per-fluoromorpholino, or N-(trifluoromethyl)per-fluoropiperazino;

$R^3$ represents

hydrogen, or

methyl; and

$R^4$ represents

phenyl substituted with

(1) a p-nitro group, or

(2) two to five independently selected $R^5$ groups, where $R^5$ is bromo, chloro, or fluoro, or

(3) two independently selected $R^6$ groups, where $R^6$ is iodo, nitro, cyano, trifluoromethyl, fluoromethyl, fluorosulfonyl, methylsulfonyl,

ethylsulfonyl, carbomethoxy, or carboethoxy, or

(4) two groups, including one $R^5$ group and one $R^6$ group, or

(5) two groups, including one methyl group and one $R^5$ or nitro or,

(6) two groups, including the methyl group and one fluorosulfonyl group, or

(7) three groups, including two independently selected $R^5$ groups and one $R^6$ group, or

(8) three groups, including two independently selected $R^6$ groups and one $R^5$ group, or

(9) three groups including two nitro groups and one trifluoromethyl group, or

(10) three groups including two nitro groups at the 2- and 4-positions and a $C_1$-$C_4$ alkoxy or $C_1$-$C_4$ alkylthio group at the 3- or 5-position, or

(11) two groups including a thiocyanato group and a group selected from $R^5$, iodo, and nitro, or

$R^4$ represents

naphthyl substituted with two or three independently selected $R^5$ or $R^6$ groups, or

$R^4$ represents

5-nitro-2-pyridyl;

and the sodium, potassium, and ammonium salts thereof, wherein ammonium is of the following formula

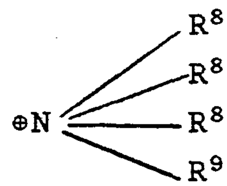

wherein each $R^8$ independently represents alkyl of $C_1$-$C_{20}$, benzyl, 2-hydroxyethyl, 2-hydroxypropyl, or 3-hydroxypropyl; and $R^9$ represents hydrogen or $R^8$, the total number of carbon atoms in all $R^8$ and $R^9$ moieties being from 12 to 60, except that when one or more $R^8$ groups are 2-hydroxyethyl, 2-hydroxypropyl, or 3-hydroxypropyl, the total number of carbon atoms in all $R^8$ and $R^9$ moieties can be from 6 to 60.

## Synthesis

The present compounds are prepared in conventional procedures for the preparation of carboxanilides. A preferred method is the reaction of the appropriate alkanoyl halide:

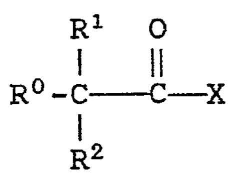 or 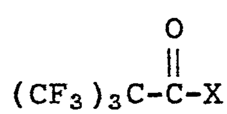 or 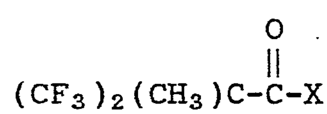

with the desired aniline, 1-aminonaphthalene, or 2-amino-5-nitropyridine, of the formula

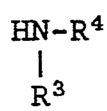 .

Preferably the halide is fluoride. In carrying out this reaction, the reactants are combined in a reaction solvent. Various solvents can be employed, including toluene, acetonitrile, diethyl ether, tetrahydrofuran, and halogenated solvents such as methylene chloride. In general, diethyl ether and halogenated solvents are preferred. A halogenated solvent can sometimes serve as the solvent in a "one-pot" reaction to make the aniline starting material which is thereafter converted to the final product of the invention. In other particulars, the reaction is conventional. An HF acceptor is provided to the reaction mixture; typically triethylamine is used. The reaction consumes the reactants and the HF acceptor in equimolar amounts. The reaction goes forward over a wide temperature range, such as from 10 to 110°C; however, the reaction is most conveniently carried out at temperatures of about 20° to 70°C. Workup of the reaction mixture to isolate the product is carried out in conventional procedures.

The alkanoyl halides employed as starting materials in this reaction route are generally prepared by electrochemical fluorination and therefore often contain isomers. In view of the requirement of the present invention for branching alpha to the carbonyl, it is often desirable to purify the anilide products to remove straight-chain isomers. It has been found that this can generally be achieved by selective hydrolysis of the straight-chain carboxanilides, and separation of the water soluble K or Na salt of the branched chain carboxanilide from the precipitated aniline. This is illustrated by Examples 2, 3, 8, and 13 below.

An alternate procedure is available for the preparation of those compounds of formula I wherein $R^0$ represents fluoro, both $R^1$ and $R^2$ represent trifluoromethyl, and $R^3$ represents hydrogen; however, this procedure is less favored than the proceding one. The procedure involves the reaction of an olefin, hexafluoropropene, and an isocyanate in the presence of a fluoride such as KF, NaF, or cesium fluoride:

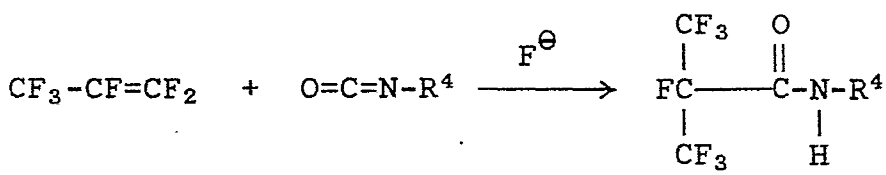

The reaction is carried out in a reaction solvent such as DMF, DMSO, or diglyme, under pressure of 5-40 psig. The reaction goes forward over a range of temperatures, such as from 30 to 70°C. The reaction requires one mole of isocyanate, three or more moles of KF, NaF, or cesium fluoride, and three to ten or more moles of hexafluoropropene. Workup is conventional.

Those compounds of the present invention in which $R^3$ represents methyl are preferably prepared from the corresponding $R^3$=hydrogen compounds. This methylation reaction is carried out in any of the known conventional methods for methylation. Typically, methyl iodide is employed as the reagent, and the reaction is carried out in a suitable solvent, such as acetone, with a base such as potassium carbonate. Equimolar amounts of the reactants are consumed, but the methyl iodide is preferably used in excess. The reaction goes forward over a wide range of temperatures, but is most conveniently

carried out at room temperatures of about 25 to 35°C. Workup is by conventional procedures.

The present invention also includes salts of the parent compounds. These salts are prepared in entirely conventional methods. The sodium and potassium salts are prepared by reacting the corresponding parent compounds with sodium or potassium hydroxide; the ammonium salts can be obtained by reacting the parent compound with a compound of the formula 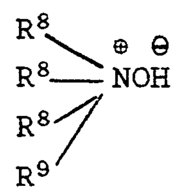 , or by reacting a sodium salt of a present compound with

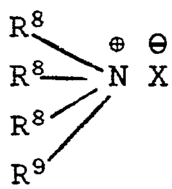 (where X = Br, Cl, or F).

Accordingly, the invention also provides a process for preparing an alkanoyl anilide of the formula (I) (II) or (III), as defined above, or a sodium, potassium or ammonium salt thereof, which comprises

(a) reacting an amine of the formula $HNR^3R^4$, wherein $R^3$ and $R^4$ are as defined above, with an alkanoyl halide of the formula $(CR^0R^1R^2)COX$, $(CF_3)_3CCOX$, or $(CF_3)_2(CH_3)CCOX$, or

(b) reacting an olefin of the formula $CF_3$-CF=$CF_2$ with an isocyanate of the formula O=C=$NR^4$ in the presence of a fluoride to produce a compound of formula (I) wherein $R^0$ is fluoro, $R^1$ and $R^2$ are $CF_3$ and $R^3$ is hydrogen, or

(c) methylating a compound of formula (I), (II) or (III) wherein $R^3$ is hydrogen to provide the corresponding compound wherein $R^3$ is methyl, and

(d) optionally salifying the compound to provide the sodium, potassium, or $\oplus NR^8R^8R^8R^9$ salt thereof.

The synthesis of the present compounds is further taught by the following illustrative examples.

## Example 1

2'-Bromo-4'-nitro-2,3,3,3-tetrafluoro-2-(trifluoromethyl)propionanilide (olefin process)

Potassium fluoride (23 grams; 0.4 mole) which was dried by heating strongly with a bunsen burner in a porcelain crucible and subsequently powdered, was added to 200 ml of a DMF solution of 2-bromo-4-nitrophenyl isocyanate (6 grams; 0.025 mole). The mixture was placed in a pressure vessel, purged with a small stream of hexafluoropropene, and heated to 70°C while adding hexafluoropropene from a pre-weighed supply cylinder at 10-20 psig. A pressure drop occurred as the gas reacted, and the remaining hexafluoropropene was added intermittently until the supply cylinder was empty; heating was continued at 65-70°C for 2½ hours with

pressure stabilized at 5 psig. The reaction vessel was then cooled and the solution poured off and extracted with hexane. The DMF solution was poured into water and filtered. The solid products were taken up in chloroform, dried over sodium sulfate, filtered, evaporated, and chromatographed on silica gel with ethyl acetate/hexane (1:5). The front-running product was collected; H-NMR of this product indicated the desired product. The material was then chromatographed on silica gel with gradient elution from 100% hexane to 100% ethyl acetate.

The foregoing procedures yielded a waxy solid, m.p. 57-59°C, yield 1.65 grams (16%). The identity of the product was confirmed by MS, H-NMR, and $F^{19}$-NMR.

Analysis calculated for $C_{10}H_4BrF_7N_2O_3$

Theory: C, 29.08; H, 0.98; N, 6.78;

Found: C, 29.31; H, 0.83; N, 6.58.

## Example 2

2'-Bromo-4'-nitro-2,3,3,3-tetrafluoro-2-(trifluoromethyl)propionanilide (alkanoyl fluoride process, with isomer purification)

2-Bromo-4-nitroaniline (141 grams; 0.65 mole) was dissolved in 3.5 liters of diethyl ether, dried over sodium sulfate, and filtered. Triethylamine (71 grams; 0.070 mole) was added with stirring. A mixture of 2,2,3,3,4,4,4-heptafluorobutyryl fluoride, 2,3,3,3-tetrafluoro-2-(trifluoromethyl)propionyl fluoride, HF, and inert gases were employed. The mixture was believed to contain 70% of chemically active acyl fluorides; 100

grams of this mixture were add under a dry ice/acetone condensor. The reaction mixture was left to stand overnight (about 17 hours) and an additional amount of the mixture was added to a total of 210 grams (0.70 mole).

Water was added to the reaction mixture, then dilute ice/HCl until the pH of the aqueous layer was acidic. The water layer was decanted off. The diethyl ether layer was dried over magnesium sulfate and the solvent evaporated under vacuum.

The solid residue was taken up in 800 ml of ethanol, and treated with potassium carbonate at room temperature, with magnetic stirring. Sixty-eight grams of $K_2CO_3$ was added initially, followed by 23 g more after 24 hours and 12 g more after 48 hours and stirred for a total of 64 hours. The ethanol was evaporated under vacuum, and the solids were triturated three times with aqueous potassium carbonate. The solution was filtered and the filtrate was acidified below 25°C with HCl. The product was filtered, washed with water, dissolved in methylene chloride, dried, and evaporated. The gas chromatogram of the product indicated less than 1% straight-chain isomer, m.p., 66°C, yield, 178 grams (66%).

Analysis calculated for $C_{10}H_4BrF_7N_2O_3$

Theory: C, 29.08; H, 0.98; N, 6.78;

Found: C, 29.30; H, 0.92; N, 6.99.

## Example 3

2'-Bromo-4'-nitro-2,3,3,3-tetrafluoro-2-(trifluoromethyl)propionanilide (alkanoyl fluoride process, with isomer purification)

2-Bromo-4-nitroaniline (35.84 grams; 0.16 mole), triethylamine (21.15 grams; 0.21 mole), and 250 ml of tetrahydrofuran were added to a 500 ml roundbottom flask equipped with a dry ice/acetone reflux condenser and a gas inlet. The vessel was purged with nitrogen. The same mixture of acyl fluorides used in Example 2 was employed as the reactant in the present example (54.60 grams; 0.18 mole). This mixture was added at a rate allowing a slow reflux. The addition required approximately 90 minutes and the resulting brown solution was stirred for about an hour after the addition was complete.

The reaction mixture was then washed with 60 ml of a 50/50 mixture of water/saturated sodium chloride solution and 40 ml of saturated sodium chloride solution. The aqueous layers were discarded.

One hundred fifty milliliters of 1N sodium hydroxide was then added to the organic layer and 90 ml of volatile material was distilled off at atmospheric pressure. Another 100 ml of 1N sodium hydroxide was added to the solution, and the resulting solution was distilled until the head temperature rose above the boiling point of tetrahydrofuran, 66°C. The pot temperature was held constant until HPLC confirmed the

hydrolysis of the straight-chain product by the basic solution. The resulting aniline precipitated out at this time and was removed by filtration.

The filtrate was allowed to cool, then washed twice, with 150 ml and 100 ml of methylene chloride. The aqueous layer was separated and placed under vacuum to remove any residual organic solvents. The solution was then placed in an ice bath and the pH was lowered to 7 with concentrated hydrochloric acid. The desired 2'-bromo-4'-nitro-2,3,3,3-tetrafluoro-2-(trifluoromethyl)propionanilide precipitated and was separated by filtration, washed with distilled water, and dried overnight in vacuo at room temperature, 42.95 grams (86.0%) in 99.5% purity by HPLC.

Other compounds of the present invention were prepared by the alkanoyl fluoride procedure except where noted otherwise. Preparations by the alkanoyl fluoride process were isolated as isomer mixtures except as noted in Examples 8 and 13.

These other compounds of the present invention are listed in the following Examples. In each example, the identity of the product was confirmed by H-NMR. The percentage of the desired branched chain isomer in the product, as determined by $F^{19}$-NMR, is also reported.

## Example 4

2'-Cyano-4'-nitro-2,3,3,3-tetrafluoro-2-(trifluoromethyl)propionanilide, m.p., 100-101°C, yield 50% (72% branched isomer).

Analysis calculated for $C_{11}H_4F_7N_3O_3$

Theory: C, 36.79; H, 1.12; N, 11.70;

Found: C, 37.05; H, 1.22; N, 11.89.

## Example 5

2'-Methyl-4'-nitro-2,3,3,3-tetrafluoro-2-(trifluoromethyl)propionanilide, m.p., 120-122°C, yield 22% (93% branched isomer).

Analysis calculated for $C_{11}H_7F_7N_2O_3$

Theory: C, 37.95; H, 2.03; N, 8.05;

Found: C, 38.10; H, 1.95; N, 8.26.

## Example 6

2'-(trifluoromethyl)-4'-nitro-2,3,3,3-tetrafluoro-2-(trifluoromethyl)propionanilide, m.p., 53-54°C, yield 15% (>95% branched isomer).

Analysis calculated for $C_{11}H_4F_{10}N_2O_3$

Theory: C, 32.85; H, 1.00; N, 6.97;

Found: C, 32.79; H, 1.15; N, 7.01.

Example 7

2',3',4',5',6',2,3,3,3-Nonafluoro-2-(trifluoromethyl)propionanilide, m.p., 137-142°C, yield 85% (60% branched isomer).

Analysis calculated for $C_{10}HF_{12}NO$

Theory: C, 31.68; H, 0.27; N, 3.69;

Found: C, 31.42; H, 0.23; N, 3.89.

Example 8

2'-Chloro-4'-nitro-2,3,3,3-tetrafluoro-2-(trifluoromethyl)propionanilide, m.p., 52-53°C, yield 94% (86% branched isomer).

Analysis calculated for $C_{10}H_4ClF_7N_2O_3$

Theory: C, 32.59; H, 1.09; N, 7.60;

Found: C, 32.82; H, 0.97; N, 7.63.

This product was purified by treatment with potassium carbonate ethanol, as follows. The product (4 grams; 0.0108 mole) was dissolved in 20 ml of 95% ethanol and potassium carbonate (1.1 gram; 0.008 mole) was added. The mixture was maintained for 5 days at room temperature. The ethanol was then evaporated off at room temperature. Potassium carbonate (1.1 gram) in 50 ml of water was added at 40°C with vigorous stirring. The mixture was filtered and washed with 40°C water. The filtrate was cooled, acidified with HCl, filtered, and air dried, yielding 2.8 grams (70% yield)

of purified product melting at 67°C. The gas chromatogram and $F^{19}$-NMR confirmed that the product was >99% branched isomer.

Example 9

2',3',4',6',2,3,3,3-Octafluoro-2-(trifluoromethyl)propionanilide, m.p., 104-108°C, yield 31% (78% branched isomer).

Analysis calculated for $C_{10}H_2F_{11}NO$.
Theory: C, 33.26; H, 0.56; N, 3.88;
Found: C, 33.54; H, 0.78; N, 3.94.

Example 10

2',4',5'-Trichloro-2,3,3,3-tetrafluoro-2-(trifluoromethyl)propionanilide, m.p., 69-72°C, yield 55% (87% branched isomer).

Analysis calculated for $C_{10}H_3Cl_3F_7NO$
Theory: C, 30.60; H, 0.77; N, 3.57;
Found: C, 30.69; H, 1.01; N, 3.62.

Another preparation of the same compound was made, m.p., 80-81°C, yield 67% (91% branched isomer).
Found: C, 30.47; H, 1.04; N, 3.43.

Example 11

2',3',5',6',2,3,3,3-Octafluoro-2-(trifluoromethyl)propionanilide, m.p., 94-99°C, yield 69% (82% branched isomer).

Analysis calculated for $C_{10}H_2F_{11}NO$
Theory: C, 33.26; H, 0.56; N, 3.88;
Found: C, 33.06; H, 0.34; N, 4.00.

Example 12

2',3',4',5'-Tetrachloro-2,3,3,3-tetrafluoro-2-(trifluoromethyl)propionanilide, m.p., 82-83°C, yield 18% (95% branched isomer).

Analysis calculated for $C_{10}H_2Cl_4F_7NO$
Theory: C, 28.13; H, 0.47; N, 3.28;
Found: C, 28.35; H, 0.52; N, 3.30.

Example 13

2',5'-Dichloro-4'-nitro-2,3,3,3-tetrafluoro-2-(trifluoromethyl)propionanilide was prepared by the olefin procedure, m.p. 80-82°C, yield 12% (>99% branched isomer).

Analysis calculated for $C_{10}H_3Cl_2F_7N_2O_3$
Theory: C, 29.80; H, 0.75; N, 6.95;
Found: C, 29.90; H, 0.53; N, 6.98.

2',5'-Dichloro-4'-nitro-2,3,3,3-tetrafluoro-2-(trifluoromethyl)propionanilide was also prepared by the alkanoyl fluoride procedure, m.p., 81-83°C, yield 47% (94% branched isomer). Microanalysis showed

Found: C, 30.07; H, 0.53; N, 6.92.

This product was purified by treatment with potassium carbonate, as described above in Example 8, m.p. 83-85°C, yield 82% of >99% branched isomer.

## Example 14

2',3'-Dichloro-2,3,3,3-tetrafluoro-2-(trifluoromethyl)propionanilide, m.p., 71-73°C, yield 32% (99.4% branched isomer).

Analysis calculated for $C_{10}H_4Cl_2F_7NO$

Theory: C, 33.55; H, 1.13; N, 3.91;

Found: C, 33.59; H, 1.25; N, 3.95.

## Example 15

N-(5-Nitro-2-pyridyl)-2,3,3,3-tetrafluoro-2-(trifluoromethyl)propionamide, m.p., 75-77°C, yield 42% (97% branched isomer).

Analysis calculated for $C_9H_4F_7N_3O_3$

Theory: C, 32.26; H, 1.20; N, 12.54;

Found: C, 32.19; H, 1.00; N, 12.45.

Example 16

2',6'-Dichloro-4'-nitro-2,3,3,3-tetrafluoro-2-(trifluoromethyl)propionanilide, m.p., 128-134°C, yield 13% (59% branched isomer).

Analysis calculated for $C_{10}H_3Cl_2F_7N_2O_3$
Theory: C, 29.80; H, 0.75; N, 6.95;
Found: C, 29.84; H, 0.97; N, 7.09.

Example 17

2'-Methyl-4'-nitro-5'-chloro-2,3,3,3-tetrafluoro-2-(trifluoromethyl)propionanilide, m.p., 114-116°C, yield 53% (91% branched isomer).

Analysis calculated for $C_{11}H_6ClF_7N_2O_3$
Theory: C, 34.53; H, 1.58; N, 7.32;
Found: C, 34.40; H, 1.61; N, 7.31.

Example 18

2',4',6'-Trichloro-2,3,3,3-tetrafluoro-2-(trifluoromethyl)propionanilide, m.p., 133-135°C, yield 33% (87% branched isomer).

Analysis calculated for $C_{10}H_3Cl_3F_7NO$
Theory: C, 30.60; H, 0.77; N, 3.57;
Found: C, 30.47; H, 0.92; N, 3.47.

## Example 19

3',4',5'-Trichloro-2,3,3,3-tetrafluoro-2-(trifluoromethyl)propionanilide, m.p., 145-146°C, yield 38% (95% branched isomer).

Analysis calculated for $C_{10}H_3Cl_3F_7NO$

Theory: C, 30.60; H, 0.77; N, 3.57;

Found: C, 30.82; H, 1.06; N, 3.50.

## Example 20

2'-Chloro-5'-nitro-2,3,3,3-tetrafluoro-2-(trifluoromethyl)propionanilide, m.p., 68-70°C, yield 35% (84% branched isomer).

Analysis calculated for $C_{10}H_4ClF_7N_2O_3$

Theory: C, 32.59; H, 1.09; N, 7.60;

Found: C, 32.64; H, 1.33; N, 7.49.

## Example 21

2',4'-Dinitro-2,3,3,3-tetrafluoro-2-(trifluoromethyl)propionanilide, m.p., 50-51°C, yield 21% (85% branched isomer).

Analysis calculated for $C_{10}H_4F_7N_3O_5$

Theory: C, 31.68; H, 1.06; N, 11.08;

Found: C, 31.42; H, 1.15; N, 10.84.

## Example 22

2'-Iodo-4'-nitro-2,3,3,3-tetrafluoro-2-(trifluoromethyl)propionanilide, m.p., 107-110°C, yield 8% (39% branched isomer).

Analysis calculated for $C_{10}H_4IF_7N_2O_3$

Theory: C, 26.11; H, 0.88; N, 6.09;

Found: C, 26.28; H, 1.11; N, 5.90.

## Example 23

2'-Cyano-4'-chloro-2,3,3,3-tetrafluoro-2-(trifluoromethyl)propionanilide, m.p., 90-91°C, yield 5% (91% branched isomer).

Analysis calculated for $C_{11}H_4ClF_7N_2O$

Theory: C, 37.90; H, 1.16; N, 8.04;

Found: C, 38.08; H, 1.10; N, 7.84.

## Example 24

2'-Bromo-4'-nitro-2,3,3,3-tetrafluoro-2-(trifluoromethyl)propionanilide, tetra-n-propylammonium salt

2'-Bromo-4'-nitro-2,3,3,3-tetrafluoro-2-(trifluoromethyl)propionanilide, (20 grams of material free of straight-chain isomer; 0.048 mole) was dissolved in 200 ml of acetone. The solution was maintained at room temperature while 2N sodium hydroxide (25 ml; 0.05 mole) and tetra-n-propylammonium bromide (13.5

grams; 0.051 mole) were added. The reaction mixture was stirred for 45 minutes, evaporated at room temperature, and partitioned between methylene chloride/water. The organic phase was washed twice with water, dried over sodium sulfate, and evaporated to dryness, yielding 28.7 grams of the intended salt (99% yield). The identity of the product was confirmed by H-NMR. The product solidified upon standing, m.p., 57-65°C.

Analysis calculated for $C_{22}H_{31}BrF_7N_3O_3$

Theory: C, 44.16; H, 5.22; N, 7.02;

Found: C, 44.14; H, 5.05; N, 6.80.

A second preparation was conducted similarly, and the identity of the product was confirmed by H-NMR. The yield was 9.2 grams (91%), m.p. 72-75°C.

Found: C, 43.88; H, 4.50; N, 6.83.

A third preparation was similarly conducted, except the starting anilide was only 86% of the desired 2'-bromo-4'-nitro-2,3,3,3-tetrafluoro-2-(trifluoromethyl)propionanilide, the remaining 14% being the corresponding straight-chain isomer. The product of this reaction was an oil. The identity was confirmed by H-NMR.

Found: C, 44.02; H, 5.07; N, 7.02.

Other salts were prepared in similar manner and are reported below. In each preparation, the identity of the product was confirmed by H-NMR. The starting anilide in these preparations, and therefore

the corresponding salt as well, was either essentially pure 2'-bromo-4'-nitro-2,3,3,3-tetrafluoro-2-(trifluoromethyl)propionanilide (referred to as "isomer pure") or a mixture of 86% of this compound and 14% of the corresponding straight-chain isomer (referred to as "86% isomer").

## Example 25

2'-Bromo-4'-nitro-2,3,3,3-tetrafluoro-2-(trifluoromethyl)propionanilide, tetraethylammonium salt, monohydrate, m.p., 112°C, yield 60% (isomer pure).

Analysis calculated for $C_{18}H_{25}BrF_7N_3O_4$

Theory: C, 38.59; H, 4.50; N, 7.50;

Found: C, 38.85; H, 4.25; N, 7.48.

## Example 26

2'-Bromo-4'-nitro-2,3,3,3-tetrafluoro-2-(trifluoromethyl)propionanilide, tetra-n-butylammonium salt, monohydrate, m.p., 78-80°C, yield 66% (isomer pure).

Analysis calculated for $C_{26}H_{41}BrF_7N_3O_4$

Theory: C, 46.44; H, 6.14; N, 6.25;

Found: C, 46.66; H, 6.29; N, 6.12.

## Example 27

2'-Bromo-4'-nitro-2,3,3,3-tetrafluoro-2-(trifluoromethyl)propionanilide, tetra-n-pentylammonium salt, monohydrate.

Preparation #1, m.p., 55-57°C, yield 80% (isomer pure).

Analysis calculated for $C_{30}H_{49}BrF_7N_3O_4$

Theory: C, 49.45; H, 6.73; N, 5.77;

Found: C, 50.80; H, 6.93; N, 5.69.

F84-1N9-277

Preparation #2, m.p., 54-56°C, yield 76% (86% isomer)

## Example 28

2'-Bromo-4'-nitro-2,3,3,3-tetrafluoro-2-(trifluoromethyl)propionanilide, tetra-n-heptylammonium salt, monohydrate, an oil, yield 50% (isomer pure).

Analysis calculated for $C_{38}H_{65}BrF_7N_3O_4$

Theory: C, 54.28; H, 7.79; N, 5.00;

Theory*: C, 54.47; H, 7.66; N, 5.11;

Found: C, 56.27; H, 8.68; N, 4.59.

*=non hydrate

## Example 29

2'-Bromo-4'-nitro-2,3,3,3-tetrafluoro-2-(trifluoromethyl)propionanilide, methyltri-n-octylammonium salt, monohydrate, an oil, yield 70% (86% isomer).

Analysis calculated for $C_{35}H_{59}BrF_7N_3O_4$
Theory: C, 52.11; H, 7.20; N, 5.36;
Found: C, 52.63; H, 7.39; N, 5.26.

## Example 30

2'-Bromo-4'-nitro-2,3,3,3-tetrafluoro-2-(trifluoromethyl)propionanilide, methyltris($C_8$-$C_{10}$)-ammonium salt, monohydrate, an oil, yield 60% (86% isomer).

## Example 31

2'-Bromo-4'-nitro-2,3,3,3-tetrafluoro-2-(trifluoromethyl)propionanilide, hexadecyltrimethylammonium salt, monohydrate.

Preparation #1, an oil, yield 48% (isomer pure).

Analysis calculated for $C_{29}H_{47}BrF_7N_3O_4$
Theory: C, 48.74; H, 6.63; N, 5.88;
Found: C, 49.03; H, 6.39; N, 5.78.

Preparation #2, an oil, yield 6% (86% isomer).
Found: C, 47.54; H, 6.09; N, 5.90.

Example 32

2'-Bromo-4'-nitro-2,3,3,3-tetrafluoro-2-(trifluoromethyl)propionanilide, octadecyltrimethylammonium salt, m.p., 45-48°C, yield 52% (86% isomer).

Analysis calculated for $C_{31}H_{49}BrF_7N_3O_3$

Theory: C, 51.38; H, 6.76; N, 5.80;

Found: C, 52.03; H, 6.67; N, 5.67.

Example 33

2'-Bromo-4'-nitro-2,3,3,3-tetrafluoro-2-(trifluoromethyl)propionanilide, benzyltri-n-butylammonium salt, monohydrate, an oil, yield 14% (86% isomer).

Analysis calculated for $C_{29}H_{39}BrF_7N_3O_4$

Theory: C, 49.30; H, 5.56; N, 5.95;

Found: C, 50.21; H, 5.96; N, 5.64.

Example 34

2'-Bromo-4'-nitro-2,3,3,3-tetrafluoro-2-(trifluoromethyl)propionanilide, dimethylbis($C_{18}$-$C_{22}$)-ammonium salt, monohydrate, m.p., 65-70°C, yield 48% (86% isomer).

Example 35

2'-Bromo-4'-nitro-2,3,3,3-tetrafluoro-2-(trifluoromethyl)propionanilide, dimethylbis($C_{10}$-$C_{18}$)-ammonium salt, an oil, yield 42% (isomer pure).

Example 36

2'-Bromo-4'-nitro-2,3,3,3-tetrafluoro-2-(trifluoromethyl)propionanilide, dimethylbis($C_{14}$-$C_{18}$)-ammonium salt, monohydrate, an oil, yield 45% (86% isomer).

Example 37

2'-Bromo-4'-nitro-2,3,3,3-tetrafluoro-2-(trifluoromethyl)propionanilide, tributylammonium salt, m.p., 58-60°C, yield 87% (isomer pure).

Analysis calculated for $C_{22}H_{31}BrF_7N_2O_3$

Theory: C, 44.15; H, 5.18; N, 7.02;

Found: C, 43.27; H, 3.47; N, 6.96.

Except where noted otherwise, the following additional examples were prepared by the foregoing procedures.

Example 38

4'-Nitro-2,3,3,3-tetrafluoro-2-(trifluoromethyl)propionanilide, m.p., 112-115°C, yield 60% (85% branched isomer).

Analysis calculated for $C_{10}H_5F_7N_2O_3$

Theory: C, 35.95; H, 1.51; N, 8.38;

Found: C, 36.21; H, 1.50; N, 8.39.

Example 39

2'-Chloro-5'-(fluorosulfonyl)-2,3,3,3-tetrafluoro-2-(trifluoromethyl)propionanilide, m.p., 63-65°C, yield 33% (87% branched isomer).

Analysis calculated for $C_{10}H_4ClF_8NO_3S$
Theory: C, 29.61; H, 0.99; N, 3.45;
Found: C, 29.88; H, 0.98; N, 3.27.

Example 40

N-(4-Nitro-1-naphthyl)-2,3,3,3-tetrafluoro-2-(trifluoromethyl)propionamide, m.p., 127-128°C, yield 5% (80% branched isomer).

Analysis calculated for $C_{14}H_7F_7N_2O_3$
Theory: C, 43.77; H, 1.84; N, 7.29;
Found: C, 43.96; H, 1.82; N, 7.52.

Example 41

2'-Bromo-4'-chloro-6'-cyano-2,3,3,3-tetrafluoro-2-(trifluoromethyl)propionanilide, an oil, yield 45% (53% branched isomer).

Analysis calculated for $C_{11}H_3BrClF_7N_2O$
Theory: C, 30.91; H, 0.71; N, 6.55;
Found: C, 31.15; H, 0.99; N, 6.30.

Example 42

3'-Chloro-4'-nitro-2,3,3,3-tetrafluoro-2-(trifluoromethyl)propionanilide, m.p., 55-57°C, yield 9% (73% branched isomer).

Analysis calculated for $C_{10}H_4ClF_7N_2O_3$
Theory: C, 32.59; H, 1.09; N, 7.60;
Found: C, 32.55; H, 1.34; N, 7.30.

Example 43

2'-Bromo-4'-nitro-2,3,3,3-tetrafluoro-2-(trifluoromethyl)propionanilide was reacted with methyl iodide, in acetone and in the presence of potassium carbonate, yielding N-methyl-2'-bromo-4'-nitro-2,3,3,3-tetrafluoro-2-(trifluoromethyl)propionanilide, an oil, yield 70% (>99% branched isomer).

Analysis calculated for $C_{11}H_6BrF_7N_2O_3$
Theory: C, 30.94; H, 1.42; N, 6.56;
Found: C, 31.01; H, 1.28; N, 6.77.

Example 44

2'-Bromo-4'-nitro-6'-cyano-2,3,3,3-tetrafluoro-2-(trifluoromethyl)propionanilide, m.p., 109-111°C, yield 10% (30% branched isomer).

Analysis calculated for $C_{11}H_3BrF_7N_3O_3$
Theory: C, 30.16; H, 0.69; N, 9.59;
Found: C, 30.41; H, 0.62; N, 9.81.

Example 45

2'-Methyl-3'-nitro-2,3,3,3-tetrafluoro-2-(trifluoromethyl)propionanilide, m.p., 100-102°C, yield 55% (82% branched isomer).

Analysis calculated for $C_{11}H_7F_7N_2O_3$

Theory: C, 37.95; H, 2.03; N, 8.05;

Found: C, 37.68; H, 2.01; N, 8.01.

Example 46

2',4'-Dinitro-5'-ethoxy-2,3,3,3-tetrafluoro-2-(trifluoromethyl)propionanilide, m.p., 117-119°C, was obtained in 10% yield from the attempted purification of crude 2',4'-dinitro-5'-fluoro-2,3,3,3-tetrafluoro-2-(trifluoromethyl)propionanilide with $K_2CO_3$ in ethanol (>99% branched isomer).

Analysis calculated for $C_{12}H_8F_7N_3O_6$

Theory: C, 34.04; H, 1.89; N, 9.93;

Found: C, 34.19; H, 1.80; N, 10.15.

Example 47

2'-Bromo-4'-nitro-2,3,3,3-tetrafluoro-2-(pentafluoroethoxy)propionanilide, yield 51%, in mixture with

2'-bromo-4'-nitro-2,2,3,3-tetrafluoro-3-(pentafluoroethoxy)propionanilide and an unidentified third component.

Analysis calculated for $C_{11}H_4BrF_9N_2O_4$

Theory: C, 27.56; H, 0.84; N, 5.85;

Found: C, 27.46; H, 0.91; N, 5.75.

Example 48

The mixture from the preceding example was purified by treatment with $K_2CO_3$/ethanol, yielding 2'-bromo-4'-nitro-2,3,3,3-tetrafluoro-2-(pentafluoroethoxy)propionanilide, m.p., 49°C, yield 50% (14% unidentified third component).

Analysis calculated for $C_{11}H_4BrF_9N_2O_4$

Theory: C, 27.58; H, 0.84; N, 5.85;

Found: C, 27.80; H, 1.04; N, 6.08.

Example 49

2'-Bromo-4'-nitro-2,3,3,3-tetrafluoro-2-(octafluoromorpholino)propionanilide, m.p., 50-52°C, yield 74% (>99% branched isomer).

Analysis calculated for $C_{13}H_4BrF_{12}N_3O_4$

Theory: C, 27.20; H, 0.70; N, 7.32;

Found: C, 27.42; H, 0.75; N, 7.32.

Example 50

2'-Bromo-4'-nitro-2,3,3,3-tetrafluoro-2-(heptafluoro-n-propoxy)propionanilide, m.p., 49-51°C, yield 35%. A minor component of 2'-bromo-4'-nitro-2,3,3,3-tetrafluoro-2-(nonafluoro-n-butoxy)propionanilide was detected by $F^{19}$-NMR.

Analysis calculated for $C_{12}H_4BrF_{11}N_2O_4$

Theory: C, 27.22; H, 0.76; N, 5.29;

Found: C, 26.99; H, 0.72; N, 5.55.

Example 51

2',4'-Dinitro-2,3,3,3-tetrafluoro-2-(heptafluoro-n-propoxy)propionanilide, an oil, yield 50%. A minor component of 2',4'-dinitro-2,3,3,3-tetrafluoro-2-(nonafluoro-n-butoxy)propionanilide was detected by $F^{19}$-NMR.

Analysis calculated for $C_{12}H_4F_{11}N_3O_6$

Theory: C, 29.11; H, 0.81; N, 8.49;

Found: C, 29.31; H, 1.05; N, 8.56.

Example 52

2'-Bromo-4'-nitro-2-(trifluoromethyl)-2,3,3,4,4,5,5,5-octafluorovaleranilide, m.p., 39-40°C, yield 44% (isomer pure).

Analysis calculated for $C_{12}H_4BrF_{11}N_2O_3$

Theory: C, 28.07; H, 0.78; N, 5.46;

Found: C, 28.42; H, 0.96; N, 5.67.

Example 53

2'-(Trifluoromethyl)-4'-bromo-2,3,3,3-tetrafluoro-2-(trifluoromethyl)propionanilide, m.p., 37-40°C, yield 49% (81% branched isomer).

Analysis calculated for $C_{11}H_4BrF_{10}NO$

Theory: C, 30.30; H, 0.92; N, 3.21;

Found: C, 30.07; H, 1.02; N, 3.20.

Example 54

2',5'-Dichloro-4'-nitro-2,3,3,3-tetrafluoro-2-(trifluoromethyl)propionanilide, octadecyltrimethylammonium salt monohydrate, m.p., 80-82°C, yield 99% (96% branched isomer).

Analysis calculated for $C_{31}H_{50}Cl_2F_7N_3O_4$

Theory: C, 51.81; H, 7.01; N, 5.74;

Found: C, 49.75; H, 5.56; N, 5.42.

Example 55

2',5'-Dichloro-4'-nitro-2,3,3,3-tetrafluoro-2-(trifluoromethyl)propionanilide, hexadecyltrimethylammonium salt, monohydrate, m.p., 79-81°C, yield 99% (94% branched isomer).

Analysis calculated for $C_{29}H_{46}Cl_2F_7N_3O_4$

Theory: C, 49.43; H, 6.53; N, 5.96;

Found: C, 47.65; H, 6.11; N, 7.16.

Example 56

2'-Cyano-4'-nitro-2,3,3,3-tetrafluoro-2-(heptafluoro-n-propoxy)propionanilide, m.p. 96°C, yield 17%. A minor component of 2'-cyano-4'-nitro-2,3,3,3-tetrafluoro-2-(nonafluoro-n-butoxy)propionanilide was detected by $F^{19}$-NMR.

Analysis calculated for $C_{13}H_4F_{11}N_3O_4$

Theory: C, 32.86; H, 0.85; N, 8.84;

Found: C, 33.06; H, 1.14; N, 8.69.

Example 57

2',4'-Dinitro-5'-fluoro-2,3,3,3-tetrafluoro-2-(heptafluoro-n-propoxy)propionanilide, an oil, yield 62%. A minor component of 2',4'-dinitro-5'-fluoro-2,3,3,3-tetrafluoro-2-(nonafluoro-n-butoxy)propionanilide was detected by $F^{19}$-NMR.

Analysis calculated for $C_{12}H_3F_{12}N_3O_6$

Theory: C, 28.09; H, 0.59; N, 8.19;

Found: C, 27.99; H, 0.75; N, 8.27.

Example 58

2',4'-Dinitro-2-(trifluoromethyl)-2,3,3,4,4,-5,5,5-octafluorovaleranilide, an oil, yield 13%.

Analysis calculated for $C_{12}H_4F_{11}N_3O_5$

Theory: C, 36.08; H, 0.84; N, 8.77;

Found: C, 30.36; H, 0.88; N, 8.98.

Example 59

2',4'-Dinitro-5'-fluoro-2-(trifluoromethyl)-2,3,3,4,4,5,5,5-octafluorovaleranilide, an oil, yield 58%.

Analysis calculated for $C_{12}H_3F_{12}N_3O_5$
Theory: C, 28.99; H, 0.61; N, 8.45;
Found: C, 29.20; H, 0.84; N, 8.71.

Example 60

2'-Bromo-4'-chloro-6'-cyano-2,3,3,3-tetrafluoro-2-(heptafluoro-n-propoxy)propionanilide, an oil, yield 51%. A minor component of 2'-bromo-4'-chloro-6'-cyano-2,3,3,3-tetrafluoro-2-(nonafluoro-n-butoxy)propionanilide was detected by $F^{19}$-NMR.

Analysis calculated for $C_{13}H_3BrClF_{11}N_2O_2$
Theory: C, 29.21; H, 0.61; N, 5.68;
Found: C, 29.41; H, 0.87; N, 5.48.

Example 61

2'-Bromo-4'-nitro-2,3,3,4,4,5,5,6,6,6-decafluoro-2-(pentafluoroethyl)hexananilide.

This compound was prepared from 2,3,3,4,4,5,-5,6,6,6-decafluoro-2-(pentafluoroethyl)hexanoic acid by conversion to the acid fluoride by electrochemical fluorination as follows:

In a Teflon jar equipped with a stainless steel condensor maintained at from -40 to -50°C., approximately 80 cc of commercial anhydrous HF underwent a pre-electrolysis to remove the last traces of water. An electrode pack of about 2 $in^3$ in size consisting of alternating nickel and carbon steel plates was used under a nitrogen atmosphere at a maximum current density of about 20 $ma/cm^2$ and at or below a cell voltage of 5.2 volts relative to a copper reference electrode. 4.1 g of the acid was added and approximately 0.5 amp hrs was passed. The reaction mixture was extracted with four 20 cc portions of $CFCl_3$, and the extracts were added to 1.6 g of 2-bromo-4-nitroaniline and 1.5 g of triethylamine in 25 cc of methylene chloride. The organic layer was washed with dilute HCl, dried over magnesium sulfate, evaporated, and the residue was chromatographed on silica gel with toluene to give a crude mixture whose $F^{19}$-NMR spectrum was consistent with the desired product. The crude mixture was chromatographed again with ethylacetate/hexane (1:5) to produce 100 mg of the desired anilide, an oil, in 1.6% yield.

Analysis calculated for $C_{14}H_4BrF_{15}N_2O_3$

Theory: C, 27.43; H, 0.66; N, 4.57;

Found: C, 27.56; H, 0.73; N, 4.75.

## Insecticidal Utility

As noted above, the compounds of the present invention exhibit excellent insecticidal and arachnicidal activity.

Representative compounds of the invention were tested and found to be highly active against a variety of insects and arachnids, including:

southern armyworm larve - Spodoptera eridania
two spotted spider mite - Tetranychus urticae
melon aphid - Aphis gossypii
southern corn rootworm larve - Diabrotica undecimpunctata howardi
cotton bollworm - Heliothis zea
corn leafhopper - Dalbulus malidis
black cutworm - Agrotis ipsilon
diamondback moth - Plutella xylostella
German cockroach - Blattella germanica

The compounds of Examples 1 and 36 were evaluated for the control of corn rootworm at numerous locations throughout the midwestern U. S. Observations were also made for various parameters of phytotoxicity. Each compound was formulated as a 10% granule; application rates were 0.85 - 3.4 grams/100 row feet. At most locations, each of the compounds controlled corn rootworm with no phytotoxicity. At some locations, phytotoxicity was observed, especially at higher rates. The phytotoxicity took the form of some delay in emergence, and the compound of Example 1 in addition showed crop injury on emergence, early and midseason root

injury, and midseason crop injury. The phytotoxicity was observed despite the fact that many of the trials employed post presswheel placement, therefore suggesting the need for even better separation of the present compounds from the corn seed or a safer formulation.

In European field trials at 1 - 4 kg/ha on wheat, potatoes, and sugar beets, each of the compounds controlled wireworm.

However, the compound of Example 1 exhibited crop injury to wheat and potatoes, and both compounds exhibited unacceptable crop injury to sugar beets.

Application of Example 36 to bluegrass showed no injury. Although no insecticidal efficacy data were obtained in the trial, the finding suggests that the present compounds can be used for the control of turf insects.

The compounds of the present invention are effective against organisms that attack foliage as well as organisms that live in the soil and attack roots and other underground parts of plants.

Therefore, in another embodiment, the present invention is directed to a method of inactivating an insect or arachnid which comprises applying to a locus of the insect or arachnid an effective amount of one or more of the compounds of the present invention.

Insects and arachnids against which the present method can be practiced include the various species identified above as well as many others, including, among the insects, the following

Coleoptera

Anthonomus grandis - boll weevil
Conotrachelus nenuphar - plum curculio
Curculio caryae - pecan weevil
Diabrotica spp. - rootworm and cucumber beetles
Echinocnemus squameus - rice plant weevil
Epitrix hirtipennis - tobacco flea beetle
Eutheola humilis rugiceps - sugarcane beetle
Hypera postica - alfalfa weevil
Leptinotarsa decimlineata - Colorado potato beetle
Lissorhoptrus oryzophilus - rice water weevil
Oulema oryzae - rice leaf beetle
Phyllotreta striolata - striped flea beetle
Melanotus spp. and Agriotes spp. - wireworms
Stenolophus lecontei - seedcorn beetle
Popillia japonica - Japanese beetle
Sphenophorus maidis - maize billbug
Systena blanda - palestriped flea beetle

Diptera

Contarinia sorghicola - sorghum midge
Dacus dorsalis - oriental fruit fly
Liriomyza spp. - leaf miner
Rhagoletis pomonella - apple maggot
Hylemia spp. and Delia spp. - root and seed maggots

Heteroptera

Anasa tristis - squash bug
Blissus leucopterus leucopterus - chinch bug
Euschistus servus - brown stink bug
Lygus lineolaris - tarnished plant bug

*Nezara viridula* - southern green stink bug
*Oebalus pugnax* - rice stink bug
*Pseudatomoscelis seriatus* - cotton fleahopper

Homoptera

*Clastoptera achatina* - pecan spittlebug
*Empoasca fabae* - potato leafhopper
*Eriosoma lanigerum* - wooly apple aphid
*Fiorinia theae* - tea scale
*Graminella nigrifrons* - blackfaced leafhopper
*Icerya purchasi* - cottony cushion scale
*Laodelphax striatellus* - small brown planthopper
*Lepidosaphes ulmi* - oystershell scale
*Myzus persicae* - green peach aphid
*Nephotettix cincticeps* - green rice leafhopper
*Niloparvata lugens* - brown rice planthopper
*Phylloxera devastatrix* - pecan phylloxera
*Planococcus citri* - citrus mealybug
*Psylla pyricola* - pear psylla
*Quadraspidiotus perniciosus* - San Jose scale
*Rhopalosiphum maidis* - corn leaf aphid
*Sipha flava* - yellow sugarcane aphid
*Sogatella furcifera* - whitebacked planthopper
*Spissistilus festinus* - threecornered alfalfa hopper
*Trialeurodes vaporariorum* - greenhouse whitefly
*Anuraphis maidiradicis* - corn root aphid

Hymenoptera

*Atta* spp. - leafcutter ants
*Camponotus* spp. - carpenter ants
*Dolichovespula* spp. - yellowjackets

Solenopsis invicta - red imported fire ant
Tetramorium caespitum - pavement ant
Vespidae spp. - hornets, wasps

Isoptera

Coptotermes formosanus - Formosan subterranean termite
Reticulitermes flavipes - eastern subterranean termite

Lepidoptera

Agrotis spp. and other genera - cutworms
Alabama argillacea - cotton leafworm
Anticarsia gemmatalis - velvetbean caterpillar
Buccalatrix thurberiella - cotton leafperforator
Chilo suppressalis - rice stem borer
Choristoneura fumiferana - spruce budworm
Cydia pomonella - codling moth
Elasmopalpus lignosellus - lesser cornstalk borer
Grapholita molesta - oriental fruitmoth
Heliothis virescens - tobacco budworm
Heliothis zea - cotton bollworm
Keiferia lycopersicella - tomato pinworm
Ostrinia nubilalis - European corn borer
Parnara guttata - rice skipper
Pectinophora gossypiella - pink bollworm
Pieris rapae - imported cabbageworm
Plutella xylostella - diamondback moth
Pseudoplusia includens - soybean looper
Sesamia inferens - rice swarming caterpillar
Spodoptera littoralis - Egyptian cotton leafworm
Spodoptera spp. - armyworms
Synanthedon spp. - clearwing moths

*Trichoplusia ni* - cabbage looper
*Tryporyza incertula* - yellow rice borer
*Crambus* spp. - webworms

Orthoptera
*Blatella* spp. - cockroaches
*Gryllus* spp. - field crickets
*Melanoplus* spp. - grasshoppers
*Periplaneta* spp. - cockroaches
*Scapteriscus acletus* - southern mole cricket

Thysanoptera
*Frankliniella tritici* - flower thrips
*Sericothrips variabilis* - soybean thrips
*Thrips simplex* - gladiolus thrips
*Thrips tabaci* - onion thrips

and among the arachnids, the following:

| Family | Scientific Name | Common Name |
|---|---|---|
| ACARIDAE | | |
| | *Aleurobius farinae* | Flour mite |
| | *Rhizoglyphus echinopus* | Bulb mite |
| | *Rhizoglyphus elongatus* | Elongate mite |
| | *Rhizoglyphus rhizophagus* | Root mite |
| | *Rhizoglyphus sagittatae* | Balsam root mite |
| | *Rhizoglyphus tarsalis* | Beet mite |
| ERIOPHYIDAE | | |
| | *Abacarus hystrix* | |
| | *Aceria brachytarsus* | |
| | *Aceria essigi* | Redberry mite |

| | |
|---|---|
| Aceria ficus | |
| Aceria fraxinivorus | |
| Aceria granati | |
| Aceria parapopuli | Cottonwood mite |
| Aceria sheldoni | Citrus bud mite |
| Aceria tulipae | Wheat curl mite |
| Aceria schlechtendali | Apple rust mite |
| Eriophyes convolvens | |
| Eriophyes insidiosus | |
| Eriophyes malifoliae | Apple leaf mite |
| Eriophyes padi | Plum twig gall mite |
| Eriophyes pruni | Plum leaf gall mite |
| Eriophyes pyri | Pear leaf blister mite |
| Eriophyes ramosus | Juniper mite |
| Eriophyes ribis | Currant gall mite |
| Eriophyes vitis | Grape erineum mite |
| Phyllocoptes gracilis | Blackberry mite |
| Phyllocoptruta oleivora | Citrus rust mite |
| Phytoptus ribis | |
| Trisetacus pini | Pine needle mite |
| Vasates amygdalina | David peach mite |
| Vasates cornutus | Peach silver mite |
| Vasates eurynotus | Celery rust mite |
| Vasates schlechtendali | Rusty leaf mite |

EUPODIDAE

Linopodes spp.

| | | |
|---|---|---|
| PENTHALEIDAE | | |
| | Halotydeus destrustor | Redlegged earth mite |
| | | Black sand mite |
| | Penthaleus major | Winter grain mite |
| | | Blue oat mite |
| PYEMOTIDAE | | |
| | Siteroptes cerealium | |
| TARSONEMIDAE | | |
| | Polyphagotarsonemus latus | Broad mite |
| | Steneotarsonemus pallidus | Cyclamen mite |
| TENUIPALPIDAE | | |
| | Brevipalpus californicus | California citrus mite |
| | Brevipalpus obovatus | |
| | Brevipalpus lewisi | Flat mite |
| | Tenuipalpes granati | |
| | Tenuipalpes pacificus | |
| TETRANYCHIDAE | | |
| | Bryobia arborea | Brown mite |
| | Bryobia rubrioculus | |
| | Eotetranychus coryli | |
| | Eotetranychus lewisi | Lewis spider mite |
| | Eotetranychus sexmaculatus | Sixspotted spider mite |
| | Eotetranychus weldoni | Weldon's mite |

| | |
|---|---|
| *Eotetranychus willametti* | |
| *Eutetranychus banksi* | Texas citrus mite |
| *Mediolata mali* | Apple mite |
| *Oligonychus ilicis* | Southern red mite |
| *Oligonychus pratensis* | Banks grass mite |
| *Oligonychus ununguis* | Spruce tree spider mite |
| *Panonychus citri* | Citrus red mite |
| *Panonychus ulmi* | European red mite |
| *Paratetranychus modestus* | Corn mite |
| *Paratetranychus pratensis* | Date mite |
| *Paratetranychus viridis* | Green mite |
| *Petrobia decepta* | Barley mite |
| *Schizotetranychus celarius* | Bamboo mite |
| *Schizotetranychus pratensis* | Alfalfa mite |
| *Tetranychus canadensis* | Fourspotted mite |
| *Tetranychus cinnabarinus* | |
| *Tetranychus mcdanieli* | McDaniel mite |
| *Tetranychus pacificus* | Pacific mite |
| *Tetranychus schoenei* | Schoene mite |
| *Tetranychus telarius* | Common red spider |
| *Tetranychus urticae* | Twospotted spider mite |
| *Tetranychus turkestani* | Strawberry mite |
| *Tetranychus desertorum* | Desert mite |

The present compounds are employed for the control of insects and arachnids in accordance with standard practices of the agricultural chemical industry.

Thus, the compounds, while they can be employed alone, are preferably formulated with conventional adjuvants, as described in more detail in the section below concerning Formulations. The amount of the present compounds which will provide insecticidal and/or arachnicidal activity is not critical and will vary widely, depending on the locus (foliage, soil, etc.), the susceptibility of the particular insect or arachnid, the particular compound chosen, weather and soil conditions, and the like. In general, when employing the compounds in spray formulations to be applied to foliage, concentrations of from 1 to 5000 ppm are efficacious; however, lesser concentrations such as from 1 to 100 ppm are often equally efficacious. In the case of application to soil to control soil-dwelling organisms, concentrations in the soil of from 1 ppm or less to as much as 50 ppm provide good activity. With the more active members of the series, lesser concentrations of from 1 to 10 ppm provide good activity.

Application of the compounds of the present invention is by conventional practices. Where the compounds are employed for the control of corn rootworm and other soil dwelling organisms that attack roots of plants, it is often preferred to apply a compound in a band along and including the crop row. This maximizes the protection of the crop, while minimizing the total amount of compound applied. Also, since the compounds of the present invention exhibit some phytotoxicity, it is preferred that the compounds be employed in a technique by which a pesticide is prevented from contacting seeds, for example, post presswheel placement.

## Preferred Embodiments

Although the compounds of the present invention are generally effective for the control of numerous insect and arachnid species, they have been found to be exceptionally effective for the control of the larval stage of corn rootworm (Diabrotica spp.) Therefore, a preferred embodiment of the present invention is a method of inactivating a corn rootworm larva which comprises applying to a locus of the larva an effective amount of a present compound.

In addition to this preferred embodiment in the identity of the insects or arachnids controlled by the present invention, there are preferred embodiments among the compounds of the present invention.

The preferences among compounds of all of formulae I, II, and III are

(1) a preference for $R^3$ = hydrogen,

(2) a preference for $R^4$ = substituted aryl as defined, more particularly, for $R^4$ = substituted phenyl as defined, and especially for the following substituted phenyl groups:

(a) 2,4-disubstituted phenyl of the formula

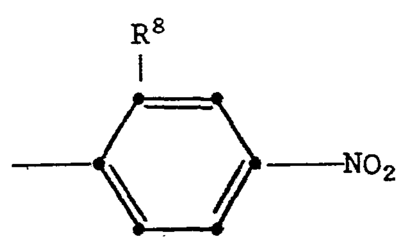

wherein $R^8$ = chloro, bromo, iodo, cyano, methyl, trifluoromethyl, or nitro; and

(b) 2,4,5(or 2,4,6)-trisubstituted phenyl of the formula

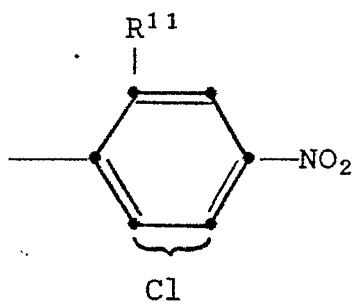

wherein $R^{11}$ = bromo, chloro, or methyl.

The preferences among compounds of formula I are

(1) $R^0$ = fluoro and

(2) each of $R^1$ and $R^2$ = $CF_3$, $C_2F_5$, or $C_3F_7$.

Among the salts of the present invention, it has been found that the ammonium salts are preferred, and among the ammonium salts, the following particular salts are especially advantageous: hexadecyltrimethylammonium, octadecyltrimethylammonium, tributylammonium, tris(2-hydroxyethyl)ammonium, tris(2 or 3-hydroxypropyl)ammonium, and dimethylbis($C_{10}$-$C_{18}$)ammonium.

## Herbicidal Activity

In addition to exhibiting activity against insects and arachnids, the present compounds also exhibit some herbicidal activity. In general, the herbicidal activity is expressed at rates higher than

those at which insecticidal and arachnicidal activity is exhibited. Therefore, the preferred insecticidal/arachnicidal utility of the present invention can generally be practiced with minimal or no phytotoxicity.

Representative compounds of the present invention were evaluated for herbicidal efficacy in standard screening tests, and it was found that the compounds typically exhibit herbicidal activity at rates of from 0.05 to 8 lbs/acre. Therefore, in another embodiment, the present invention is directed to a method for inhibiting the growth of a plant which comprises applying to the plant an effective amount of a compound of the present invention. The compounds can be applied pre-emergently for the control of germinating seeds or post-emergently for the control of existing vegetation. The compounds are preferably formulated as described below.

## Other Utilities

Compounds of the present invention have exhibited nematocidal, fungicidal, anthelmintic, and ectoparasiticidal activity. By appropriate selection of rates and methods of application, therefore, the present compounds can be employed for these other utilities. For all of these uses, the compounds are employed in conventional manners.

Accordingly, in another embodiment, the present invention is directed to a method of inhibiting a plant pathogenic fungal organism which comprises

applying to a locus of the organism an inhibiting amount of a compound of the present invention. The method is practiced in accordance with standard techniques for the use of fungicides. The compounds can be formulated as described below in the Formulations section. In general, good fungicidal efficacy can be expected at rates of 0.5-5.0 lbs/acre.

## Formulations

For any of their various uses, the compounds of the present invention are preferably formulated with a suitable agriculturally acceptable carrier. Typically such a formulation will contain from about 0.05 to about 95.0 percent by weight of the active ingredient. Examples of such compositions include sprayable formulations, such as wettable powders, aqueous suspensions and emulsifiable concentrates; and solid compositions, such as dusts, granules, and dry-flowable pellets. The compounds can also be formulated with fertilizer and applied to soil to achieve both a utility in accordance with the present invention as well as fertilization of the crop.

Sprayable formulations are in the form of concentrated compositions which can be diluted with water to form water dispersions or emulsions containing in the range from about 0.05 percent to about 10 percent of the active agent by weight. Such water dispersions or emulsions are sprayed onto plants or onto soil. The concentrated compositions may be either solids, usually known as wettable powders or dry

flowables, or liquids, usually known as emulsifiable concentrates and aqueous suspensions.

A typical wettable powder comprises an intimate mixture of an active ingredient of the invention, an inert carrier, and surfactants. The concentration of the active agent is usually from about 25 percent to about 90 percent by weight. The inert carrier is usually chosen from among the attapulgite clays, the montmorillonite clays, the diatomaceous earths, the kaolinites, or the purified silicates. Effective surfactants, comprising from about 0.5 percent to about 10 percent by weight of the wettable powder, are chosen from among the condensed naphthalenesulfonates, the alkyl sulfates and the alkyl arylethoxylates. Suspending agents, such as the sulfonated lignins, can also be added.

A typical emulsifiable concentrate comprises from about 0.1 to about 6 pounds (from about 0.045 kg to about 3.05 kg) of a compound of the invention per gallon of liquid, dissolved in a mixture of organic solvents and emulsifiers. The organic solvent is chosen with regard to its solvency and its cost. Useful solvents include the aromatics, especially the xylenes and the heavy aromatic naphthas. Hydrophilic cosolvents such as DMF, cyclohexanone, and the glycol ethers such as 2-methoxyethanol may be included. Other organic solvents may also be used, including the terpenic solvents and kerosene; methyl heptyl ketone and other high molecular weight ketones; cyclohexyl acetate and other high molecular weight esters. Suitable emulsifiers for emulsifiable concentrates are chosen from the alkylbenzenesulfonates, naphthalenesulfonates, and nonionic surfactants such as alkylphenol adducts of polyoxyethylene, and are used at similar percentages as for wettable powders.

An aqueous suspension, or flowable, is comprised of a finely ground suspension of the active ingredient dispersed in a water based system. This type of formulation is particularly useful for compounds with low water solubility. The concentration of active agent is usually from about 15 to 60 percent by weight. A typical aqueous suspension may comprise wetting and dispersing agents, antifreeze components, thickening or bulking agents, as well as water and the active ingredient.

Dust compositions containing a compound of the present invention usually contain from about 0.1 to about 50 percent by weight of the compound. Dusts are prepared by intimately mixing and finely grinding the active agent with an inert solid such as ground montmorillonite clay, attapulgite clay, talc, ground volcanic rock, kaolin clay, or other inert, relatively dense, inexpensive substances.

Solid, granular compositions are convenient for the application of compounds of this invention to the soil and will contain the active agent in an amount from about 0.1 to about 25 percent by weight. Granules comprise a compound of the invention dispersed on a granular inert carrier such as coarsely ground clay of from about 0.1 to about 3 mm particle size. The active ingredient is most conveniently applied to the clay by dissolving it in an inexpensive solvent, such as acetone, methylene chloride, xylene or other petroleum solvents, methoxy propylene glycol, or the like, and applying the solution to the sized clay in an appropriate solids mixer. The solvent is then typically removed by evaporation; however removal is not essential. Alternatively, any of the present compounds which is an oil can be sprayed, with or without heating, directly onto clay. Likewise, any of the present compounds which is a solid can be melted and then sprayed directly onto clay.

Representative formulations of the present invention are illustrated below.

Example A

10% Granule, Compound of Example 24

The compound of Example 24 was dissolved in acetone (in the ratio of 1 gram of compound per 4 grams of acetone). Propylene glycol was added and the mixture stirred, then poured over the carrier, mixed well, and air dried.

| Ingredient | Percent by Weight |
|---|---|
| Compound of Example 24 | 10 |
| Propylene glycol | 5 |
| Florex 24/48 RVM | 85 |
| | 100 |

*RVM = regular volatile material

## Example B

### 10% Granule, Compound of Example 1

The compound of Example 1 was dissolved in methylene chloride (a 12% solution), poured over the carrier, mixed well, and air dried.

| Ingredient | Percent by Weight |
|---|---|
| Compound of Example 1 | 10 |
| Florex 24/48 RVM | 90 |
| | 100 |

## Example C

### 10% Granule, Compound of Example 36

The compound of Example 36 was formulated in the same procedure as the immediately preceding example.

| Ingredient | Percent by Weight |
|---|---|
| Compound of Example 36 | 10 |
| Florex 24/48 RVM | 90 |
| | 100 |

## Example D

### 10% Granule, Compound of Example 37

The compound of Example 1 was dissolved in methylene chloride and an equimolar amount of tributylamine was slowly added to the solution to produce the salt of Example 37. The solution then containing the salt of Example 37 was poured over the carrier, mixed well, and air dried.

| Ingredient | Percent by Weight |
|---|---|
| Compound of Example 37 | 14.5 |
| Lowe's clay 30/40 mesh, Bloomfield, Missouri | 85.5 |
| | 100.0 |

## Examples E-H

### 10% Granule, Compound of Example 36

The procedure of the preceding example was used to make four 10% granular formulations from the compound of Example 1 and dimethylbis($C_{14}$-$C_{18}$)ammonium chloride, the latter estimated to be only 75% pure, yielding formulations of the compound of Example 36. The four carriers were as follows:

Oil-Dri Mississippi Grey Clay
Oil-Dri Mississippi Brown Clay
Florex Clay LVM (low volatile material)
Lowe's Oran, Missouri Clay

The composition of all four formulations was as follows.

| Ingredient | Percent by Weight |
|---|---|
| Compound of Example 36 | 11.9 |
| Carrier | 88.1 |
| | 100.0 |

Examples I and J

10% Granule, Compounds of Examples 1 and 36, Carrier Study

Various carriers were evaluated for 10% granular formulations of each of the compounds of Examples 1 and 36. Each compound was dissolved in dichloromethane, mixed, poured onto the respective carrier, mixed well, and air dried. The carriers evaluated were as follows.

Oil-Dri Mississippi Grey clay (this same clay is also referred to as Agsorb RVM-MS);

Oil-Dri Mississippi Brown clay (this same clay is also referred to as Agsorb LVM-MS);

Oil-Dri Georgia white clay - Oil Dry Corporation

Florex RVM;

Florex LVM - Floridin Company

Agsorb LVM - Oil Dry Corporation

Attapulgus RVM - Englehardt Minerals

Bentonite Granular - American Colloid

Pike's Peak clay (9-J) - General Reduction Crop.

KWK Volclay - American Colloid
Lowe's Oran, Missouri clay;
Lowe's Bloomfield, Missouri clay - Lowe's Industrial Products, Inc.

All formulations had the same composition:

| Ingredient | Percent by Weight |
| --- | --- |
| Compound of Example 1 or Example 36 | 10 |
| Carrier | 90 |
| | 100 |

Based on the foregoing formulations, Oil-Dri Mississippi Grey clay and Oil-Dri Mississippi Brown clay are preferred carriers for the parent compounds, whereas Florex LVM clay and Lowe's Oran Missouri clay are preferred carriers for the salts.

## Starting Materials

As taught above, the compounds of the present invention are prepared by the reaction of an acyl halide and an aniline, 1-aminonaphthalene, or 2-amino-5-nitropyridine:

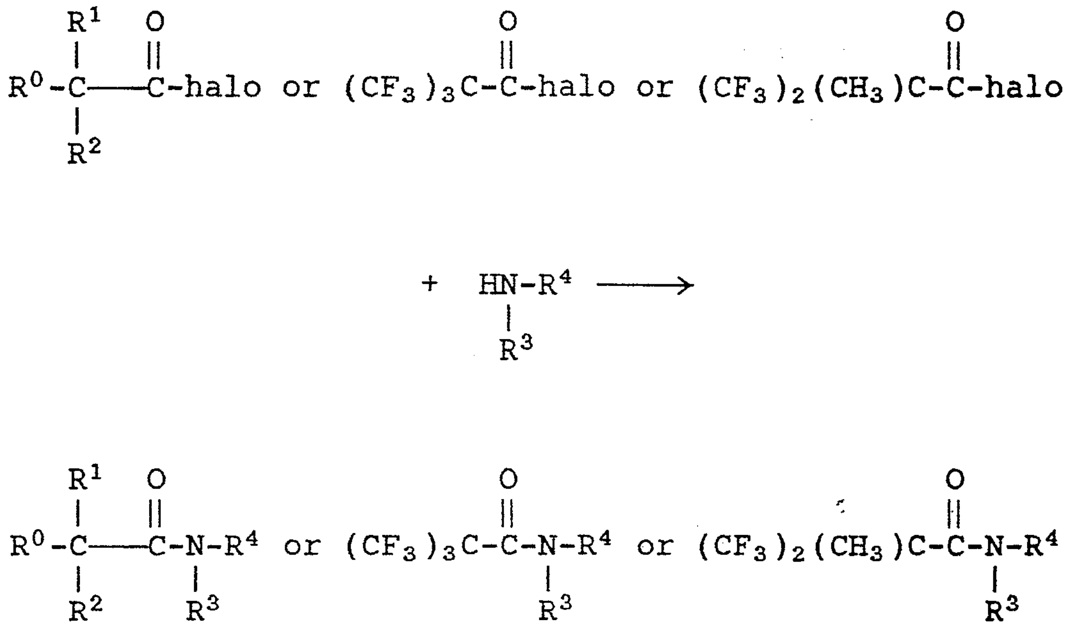


These starting materials are either known compounds or are prepared in known procedures. Essentially all of the anilines are known compounds. 1-Aminonaphthalene and 2-amino-5-nitropyridine are likewise known compounds.

Some of the alkanoyl halides are known compounds. The following are described in the literature:

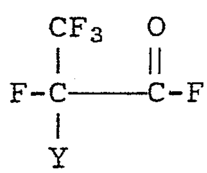
or -Cl

| Y | Reference |
|---|---|
| CN | A, B, C |
| $CF_3$ | D, E |
| n-$C_3F_7$ | F, G |

The following are also described in the literature:

$(CF_3)_3CCOF$ ——— Reference D

$(CF_3)_2BrCCOF$ ——— Reference H

$(CF_3)_2ClCCOF$ ——— Reference I

$(CF_3)_2(CH_3)CCOF$ ——— Reference J

References:

A. U.S. 4,031,124 (1977)
B. U.S. 3,933,767 (1976)
C. U.S. 3,852,326 (1974)
D. U.S. 3,113,967 (1963)
E. J. Am. Chem. Soc., 84, 4275(1962)
F. U.S. 4,172,016 (1979)
G. J. F. Chem., 12 1-25 (1978)
H. Chem. Abs. 56, 312b (1961)
I. Tetrahedron, 27 3345-3355 (1971)
J. J. F. Chem., 29 471-474 (1985)

Other of the alkanoyl halide starting materials can be prepared in analogous manner.

In addition, most of the starting alkanoyl halides can be prepared by electrochemical fluorination of precursor non-fluorinated compounds:

| Substituent on present starting material | Precursor substituent | Reference |
|---|---|---|
| F | H | Hudlicky (following table) |
| $CF_3$ | $CH_3$ | " |
| $C_2F_5$ | $C_2H_5$ | " |
| $n-C_3F_7$ | $n-C_3H_7$ | " |
| $OR_f$ | OR | A |
| $R_f$<br>/<br>N<br>\\<br>$R_f$ | $NR_2$ | B,C,D |

Similarly, many of the alkanoyl halide starting materials are conveniently prepared by electrochemical fluorination of the precursors listed above, which are already partially fluorinated.

## References:

A. U.S. 2,594,272 (1952)

B. U.K. 666,733 (1952)

C. Chem. Abs., 65, 2140g (1966)

D. Chem. Abs., 62, 16089d (1965)

A general reference on electrochemical fluorination is "Chemistry of Organic Fluorine Compounds" by M. Hudlicky (Horwood Ltd., 1976), especially page 73.

## CLAIMS

1. An alkanoyl anilide of the formulae:

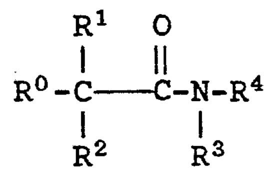

I

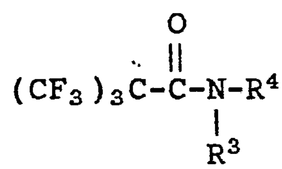

II or

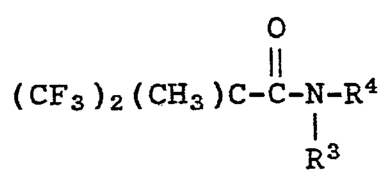

III

wherein $R^0$ represents

bromo,

chloro, or

fluoro;

$R^1$ represents

$CF_3$,

$C_2F_5$,

$C_3F_7$, or

a n-, iso-, or sec-$C_4F_9$;

$R^2$ represents

$CF_3$, $C_2F_5$, $C_3F_7$, or,

when $R^0$ represents fluoro, $R^2$ can additionally represent

$-OR_f$,

$-N(R_f)_2$,

-CN,

$-CF_2-OR_f$, or

$-CF_2-N(R_f)_2$

and each $R_f$ independently represents perfluoroloweralkyl of $C_1$-$C_3$ or, in $-N(R_f)_2$ both $R_f$ groups can be taken together with the N and constitute perfluoropyrrolidino, perfluoropiperidino, perfluoromorpholino, or N-(trifluoromethyl)perfluoropiperazino;

$R^3$ represents

hydrogen, or

methyl; and

$R^4$ represents

phenyl substituted with

(1) a *p*-nitro group, or

(2) two to five independently selected $R^5$ groups, where $R^5$ is bromo, chloro, or fluoro, or

(3) two independently selected $R^6$ groups, where $R^6$ is iodo, nitro, cyano, trifluoromethyl, fluoromethyl, fluorosulfonyl, methylsulfonyl, ethylsulfonyl, carbomethoxy, or carboethoxy, or

(4) two groups, including one $R^5$ group and one $R^6$ group, or

(5) two groups, including one methyl group and one $R^5$ or nitro or,

(6) two groups, including the methyl group and one fluorosulfonyl group, or

(7) three groups, including two independently selected $R^5$ groups and one $R^6$ group, or

(8) three groups, including two independently selected $R^6$ groups and one $R^5$ group, or

(9) three groups including two nitro groups and one trifluoromethyl group, or

(10) three groups including two nitro groups at the 2- and 4-positions and a $C_1$-$C_4$ alkoxy or $C_1$-$C_4$ alkylthio group at the 3- or 5-position, or

(11) two groups including a thiocyanato group and a group selected from $R^5$, iodo, and nitro, or

$R^4$ represents

naphthyl substituted with two or three independently selected $R^5$ or $R^6$ groups, or

$R^4$ represents

5-nitro-2-pyridyl;

and the sodium, potassium, and ammonium salts thereof, wherein ammonium is of the following formula

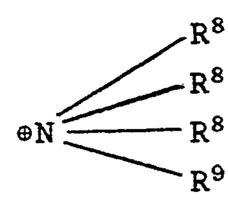

wherein each $R^8$ independently represents alkyl of $C_1$-$C_{20}$, benzyl, 2-hydroxyethyl, 2-hydroxypropyl, or

3-hydroxypropyl; and $R^9$ represents hydrogen or $R^8$, the total number of carbon atoms in all $R^8$ and $R^9$ moieties being from 12 to 60, except that when one or more $R^8$ groups are 2-hydroxyethyl, 2-hydroxypropyl, or 3-hydroxypropyl, the total number of carbon atoms in all $R^8$ and $R^9$ moieties can be from 6 to 60.

2. An alkanoyl anilide of formula (I) or (II) as claimed in claim 1, wherein $R^1$ is $CF_3$, $C_2F_5$, $C_3F_7$ or n-$C_4F_9$;

$R^2$ is $CF_3$, $C_2F_5$, $C_3F_7$ or n-$C_4F_9$, or, if $R^0$ represents fluoro, $R^2$ can additionally represent $-OR_f$, $-N(R_f)_2$, or -CN, and each $R_f$ independently represents perfluoroloweralkyl of $C_1$-$C_3$; and

$R^4$ is as defined in claim 1, provided $R^4$ is not as defined in paragraphs (6), (10) or (11), or

a sodium, potassium or $\oplus NR_8R_8R_8R_9$ salt thereof.

3. An alkanoyl anilide of formula (I) or (II) as claimed in claim 1 wherein $R^0$ is fluoro, or a sodium, potassium, or $\oplus NR_8R_8R_8R_9$ salt thereof.

4. An alkanoyl anilide of formula (I) as claimed in claim 1 wherein $R^0$ represents fluoro, $R^1$ represents $CF_3$, $C_2F_5$, or n-$C_3F_7$, and $R^3$ represents hydrogen, or a sodium, potassium, or $\oplus NR_8R_8R_8R_9$ salt thereof.

5. An alkanoyl anilide of formula (I) as claimed in claim 1, 2, 3, or 4 wherein $R^4$ represents 2-bromo-4-nitrophenyl, or a sodium, potassium, or $\oplus NR_8R_8R_8R_9$ salt thereof.

6. 2'-Bromo-4'-nitro-2,3,3,3-tetrafluoro-2-(trifluoromethyl)propionanilide or a sodium, potassium, or $\oplus NR_8R_8R_8R_9$ salt thereof.

7. An insecticidal or arachnicidal formulation comprising, as active ingredient, an alkanoyl anilide of the formula (I), (II) or (III), or a sodium, potassium, or $\oplus NR_8R_8R_8R_9$ salt thereof, as claimed in any one of claims 1-6 in combination with an agriculturally acceptable adjuvant.

8. An antifungal or herbicidal formulation comprising, as active ingredient, an alkanoyl anilide of the formula (I), (II) or (III) or a sodium, potassium, or $\oplus NR_8R_8R_8R_9$ salt thereof, as claimed in any one of claims 1-6 in combination with an agriculturally acceptable adjuvant.

9. An insecticidal or arachnicidal method which comprises applying to the locus of the insect or arachnid an effective amount of an alkanoyl anilide of formula (I), (II) or (III), or a sodium, potassium, or $\oplus NR_8R_8R_8R_9$ salt thereof as claimed in any one of claims 1-6.

10. A process for preparing an alkanoyl anilide of the formula (I) (II) or (III), as defined in claim 1, or a sodium, potassium or ammonium salt thereof, which comprises

(a) reacting an amine of the formula $HNR^3R^4$, wherein $R^3$ and $R^4$ are as defined above, with an alkanoyl halide of the formula $(CR^0R^1R^2)COX$, $(CF_3)_3CCOX$, or $(CF_3)_2(CH_3)CCOX$, or

(b) reacting an olefin of the formula $CF_3$-$CF$=$CF_2$ with an isocyanate of the formula $O$=$C$=$NR^4$ in the presence of a fluoride to produce a compound of formula (I) wherein $R^0$ is fluoro, $R^1$ and $R^2$ are $CF_3$ and $R^3$ is hydrogen, or

(c) methylating a compound of formula (I), (II) or (III) wherein $R^3$ is hydrogen to provide the corresponding compound wherein $R^3$ is methyl, and

(d) optionally salifying the compound to provide the sodium, potassium, or $^{\oplus}NR^8R^8R^8R^9$ salt thereof.

CLAIMS

1. An agrichemical formulation comprising, as active agent, an alkanoyl anilide of the formulae:

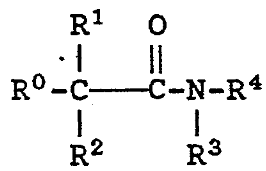

I

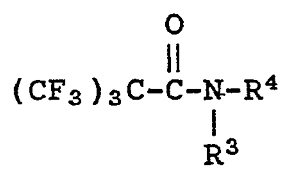

II or

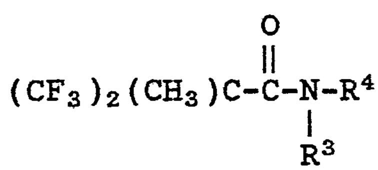

III

wherein $R^0$ represents

bromo,

chloro, or

fluoro;

$R^1$ represents

$CF_3$,

$C_2F_5$,

$C_3F_7$, or

a n-, iso-, or sec-$C_4F_9$;

$R^2$ represents

$CF_3$, $C_2F_5$, $C_3F_7$, or,

when $R^0$ represents fluoro, $R^2$ can additionally represent

$-OR_f$,

$-N(R_f)_2$,

-CN,

$-CF_2-OR_f$, or

$-CF_2-N(R_f)_2$

and each $R_f$ independently represents perfluoro-loweralkyl of $C_1$-$C_3$ or, in $-N(R_f)_2$ both $R_f$ groups can be taken together with the N and constitute perfluoropyrrolidino, perfluoropiperidino, perfluoromorpholino, or N-(trifluoromethyl)perfluoropiperazino;

$R^3$ represents

hydrogen, or

methyl; and

$R^4$ represents

phenyl substituted with

(1) a p-nitro group, or

(2) two to five independently selected $R^5$ groups, where $R^5$ is bromo, chloro, or fluoro, or

(3) two independently selected $R^6$ groups, where $R^6$ is iodo, nitro, cyano, trifluoromethyl, fluoromethyl, fluorosulfonyl, methylsulfonyl, ethylsulfonyl, carbomethoxy, or carboethoxy, or

(4) two groups, including one $R^5$ group and one $R^6$ group, or

(5) two groups, including one methyl group and one $R^5$ or nitro or,

(6) two groups, including the methyl group and one fluorosulfonyl group, or

(7) three groups, including two independently selected $R^5$ groups and one $R^6$ group, or

(8) three groups, including two independently selected $R^6$ groups and one $R^5$ group, or

(9) three groups including two nitro groups and one trifluoromethyl group, or

(10) three groups including two nitro groups at the 2- and 4-positions and a $C_1$-$C_4$ alkoxy or $C_1$-$C_4$ alkylthio group at the 3- or 5-position, or

(11) two groups including a thiocyanato group and a group selected from $R^5$, iodo, and nitro, or

$R^4$ represents

naphthyl substituted with two or three independently selected $R^5$ or $R^6$ groups, or

$R^4$ represents

5-nitro-2-pyridyl;

and the sodium, potassium, and ammonium salts thereof, wherein ammonium is of the following formula

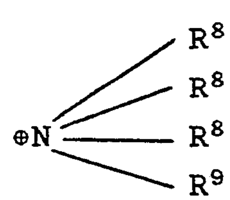

wherein each $R^8$ independently represents alkyl of $C_1$-$C_{20}$, benzyl, 2-hydroxyethyl, 2-hydroxypropyl, or

3-hydroxypropyl; and $R^9$ represents hydrogen or $R^8$, the total number of carbon atoms in all $R^8$ and $R^9$ moieties being from 12 to 60, except that when one or more $R^8$ groups are 2-hydroxyethyl, 2-hydroxypropyl, or 3-hydroxypropyl, the total number of carbon atoms in all $R^8$ and $R^9$ moieties can be from 6 to 60.

2. An agrichemical formulation of claim 1 wherein the active agent is an alkanoyl anilide of formula (I) or (II) as described in claim 1, wherein $R^1$ is $CF_3$, $C_2F_5$, $C_3F_7$ or n-$C_4F_9$;

$R^2$ is $CF_3$, $C_2F_5$, $C_3F_7$ or n-$C_4F_9$, or, if $R^0$ represents fluoro, $R^2$ can additionally represent $-OR_f$, $-N(R_f)_2$, or -CN, and each $R_f$ independently represents perfluoroloweralkyl of $C_1$-$C_3$; and

$R^4$ is as defined in claim 1, provided $R^4$ is not as defined in paragraphs (6), (10) or (11), or

a sodium, potassium or $\oplus NR_8R_8R_8R_9$ salt thereof.

3. An agrichemical formulation of claim 1 wherein the active agent is an alkanoyl anilide of formula (I) or (II) as described in claim 1 wherein $R^0$ is fluoro, or a sodium, potassium, or $\oplus NR_8R_8R_8R_9$ salt thereof.

4. An agrichemical formulation of claim 1 wherein the active agent is an alkanoyl anilide of formula (I) as described in claim 1 wherein $R^0$ represents fluoro, $R^1$ represents $CF_3$, $C_2F_5$, or n-$C_3F_7$, and $R^3$ represents hydrogen, or a sodium, potassium, or $\oplus NR_8R_8R_8R_9$ salt thereof.

5. An agrichemical formulation of claim 1 wherein the active agent is an alkanoyl anilide of

formula (I) as described in claim 1, 2, 3, or 4 wherein $R^4$ represents 2-bromo-4-nitrophenyl, or a sodium, potassium, or $\oplus NR_8R_8R_8R_9$ salt thereof.

6. An agrichemical formulation of claim 1 wherein the active agent is 2'-bromo-4'-nitro-2,3,3,3-tetrafluoro-2-(trifluoromethyl)propionanilide or a sodium, potassium, or $\oplus NR_8R_8R_8R_9$ salt thereof.

7. An insecticidal or arachnicidal formulation comprising, as active ingredient, an alkanoyl anilide of the formula (I), (II) or (III), or a sodium, potassium, or $\oplus NR_8R_8R_8R_9$ salt thereof, as defined in any one of claims 1-6 in combination with an agriculturally acceptable adjuvant.

8. An antifungal or herbicidal formulation comprising, as active ingredient, an alkanoyl anilide of the formula (I), (II) or (III) or a sodium, potassium, or $\oplus NR_8R_8R_8R_9$ salt thereof, as defined in any one of claims 1-6 in combination with an agriculturally acceptable adjuvant.

9. An insecticidal or arachnicidal method which comprises applying to the locus of the insect or arachnid an effective amount of an alkanoyl anilide of formula (I), (II) or (III), or a sodium, potassium, or $\oplus NR_8R_8R_8R_9$ salt thereof as defined in any one of claims 1-6.

10. A process for preparing an alkanoyl anilide of the formula (I) (II) or (III), as defined in claim 1, or a sodium, potassium or ammonium salt thereof, which comprises

(a) reacting an amine of the formula $HNR^3R^4$, wherein $R^3$ and $R^4$ are as defined above, with an alkanoyl halide of the formula $(CR^0R^1R^2)COX$, $(CF_3)_3CCOX$, or $(CF_3)_2(CH_3)CCOX$, or

(b) reacting an olefin of the formula $CF_3$-$CF$=$CF_2$ with an isocyanate of the formula $O$=$C$=$NR^4$ in the presence of a fluoride to produce a compound of formula (I) wherein $R^0$ is fluoro, $R^1$ and $R^2$ are $CF_3$ and $R^3$ is hydrogen, or

(c) methylating a compound of formula (I), (II) or (III) wherein $R^3$ is hydrogen to provide the corresponding compound wherein $R^3$ is methyl, and

(d) optionally salifying the compound to provide the sodium, potassium, or $^{\oplus}NR^8R^8R^8R^9$ salt thereof.